# EUROPEAN PATENT SPECIFICATION

(11) **EP 2 224 949 B1**
(45) Date of publication and mention of the grant of the patent: **17.10.2012**
(21) Application number: 08855709.5
(22) Date of filing: 01.12.2008
(51) Int. Cl.: A61P 27/00

(54) **Use of urokinase type plasminogen activator inhibitors for the treatment of corneal disorders**
Verwendung von Plasminogen-Aktivator-Inhibitoren vom Urokinase-Typ zur Behandlung von kornealen Erkrankungen
Utilisation d'inhibiteurs de l'activateur du plasminogène de type urokinase pour le traitement de troubles cornéens

(30) Priority: 30.11.2007 HU 0700769; 30.11.2007 HU 0700770
(43) Date of publication of application: 08.09.2010
(73) Proprietor: University of Debrecen, 4010 Debrecen (HU)
(72) Inventor: TÖZSÉR, József, H-4031 Debrecen (HU); BERTA, András, H-4034 Debrecen (HU); CSUTAK, Adrienne, H-4032 Debrecen (HU); MIKLÓSSY, Gabriella, H-4032 Debrecen (HU)
(74) Representative: Petho, Arpad
(86) International application number: PCT/HU2008/000143
(87) International publication number: WO 2009/068926

(56) References cited:
- US-B2- 7 179 461
- WANG ET AL: "Plasminogen activator inhibitor-1 (PAI-1) stimulates human corneal epithelial cell adhesion and migration in vitro" EXPERIMENTAL EYE RESEARCH, vol. 80, 2005, pages 1-8, XP004712444
- CEJKOVÁ ET AL: "Effects of inhibition of urokinase-type plasminogen activator (u-PA) by amiloride in the cornea and tear fluid of eyes irradiated with UVB" ACTA HISTOCHEMICA, vol. 107, 2005, pages 77-86, XP004872832
- TÖZSÉR ET AL: "The fibrinolytic system of the human tears" FIBRINOLYSIS (SUPPLEMENT 1), vol. 2, 1988, page 110, XP023288965
- BAZAN: "Cellular and molecular events in corneal wound healing: significance of lipid signalling" EXPERIMENTAL EYE RESEARCH, vol. 80, 2005, pages 453-463, XP004801645
- MISKIN ET AL: "Plasminogen activator inhibitor-1 (PAI-1) in the murine and human eye: implications for glaucoma" 2005, page 91, XP002520170 Retrieved from the Internet: URL:www.conferences.uiuc.edu/pa/program05. pdf> [retrieved on 2009-03-19]
- TÖZSÉR ET AL: "Urokinase-type plasminogen activator in rabbit tears. Comparison with human tears" EXPERIMENTAL EYE RESEARCH, vol. 51, 1990, pages 33-37, XP022969920 cited in the application

## Description

### Field of the invention

The invention concerns the use of inhibitors of the urokinase type plasminogen activator (uPA) appearing in the anterior segment of the eye, for the treatment and prevention of corneal ulcers and other disorders of the anterior segment of the eye (e.g. the cornea and the conjunctiva). The invention further concerns pharmaceutical compositions, comprising inhibitors of uPA, preferably eye drops and eye ointments. The pharmaceutical compositions according to the invention comprise PAI-2 protein or a fusion protein derivative thereof retaining uPA-inhibiting capacity, or the D-Phe-Pro-Arg-aldehyde (Ald-1). The PAI-2 protein, used according to the invention, is preferably produced through bacterial expression, as a fusion protein.

### Background of the invention

uPA is a serine protease, that can be found in the tears, and that is probably secreted by the epithelial cells of the conjunctiva and the cornea (Barlati et al., 1990, Tözsér et al., 1989, Tözsér and Berta, 1990). In a number of pathological processes, going on in the anterior segment of the eye, cellular, viral and bacterial proteases play an important role. In certain cases, due to protease overaction, harmful degradative processes occur, that may occasionally lead to blindness. In other instances low protease activity, that can be the result of unsuitable expression, or may develop due to the presence of excessive inhibitor activity, may be harmful, e.g. causing abnormal woundhealing. Similarly, uPA activities that appear in tears and in the anterior segment of the eye play a double role. On the one hand uPA, through generation of plasmin and activation of procollagenases, can cause considerable tissue destruction, the proteolytic digestion of the corneal stroma, thus may play an essential role in the development of corneal ulcers (Berman et al. 1980). In severe corneal and conjunctival inflammations, as well as in chemical bums (e.g. in lime injuries) we detected high uPA activities, that may have pathological consequences (T6zs6r et al. 1989). The epithelial cells of the ulcerating cornea and polymorphonuclear leukocytes may also contribute to the significant uPA level increase in the tear fluid (Tözsér et al. 1989).

On the other hand, uPA bound to the surface receptors of migrating epithelial cells is essential in the wound healing processes (Crippa 2007). Lack of proper urokinase activity may occur following the regently widely used laser refractive surgeries (Csutak et al. 2000), that can be prevented by using urokinase eye drops (Csutak et al. 2004). In cases with abnormal wound healing of the cornea following laser surgery stromal opacity (haze) may develop, causing a decrease in the corrected visual activities in certain cases.

Cellular, viral and bacterial proteases play a fundamental role in a number of processes going on in the anterior segment of the eye. In certain cases, due to protease overaction, pathological degrading processes occur, that may occasionally lead to blindness. Such protease overaction may appear in bacterial and in viral eye infections (e.g. conjunctivitis), as well as other inflammatory processes affecting the cornea (e.g. keratitis, ulcer).

Biochemical studies support the significance of the composition of the tears in the development of corneal ulcers. Urokinase type plasminogen activator (uPA) activity plays a crucial role in these processes. uPA is a serine protease, that can be found in the tears, that is probably secreted by epithelial cells of the conjunctiva and the cornea (Barlati et al. 1990, Tözsér and Berta 1990). uPA, through the production of plasmin and the activation of procollagenases can cause considerable tissue destruction, the proteolytic digestion of the corneal stroma, thus may play an essential role in the development of corneal ulcers (Berman et al. 1980). In severe corneal and conjunctival disorders, as well as in chemical burns (e.g. in lime injuries) we detected a significant raise in uPA activities, that may have pathological consequences (Tözsér et al. 1989). The epithelial cells of the ulcerating cornea and polymorphonuclear leukocytes may also contribute to the significant uPA level increase in the tear fluid (Tözsér et al. 1989).

The level of urokinase in the anterior segment of the eye is determined by a number of different processes. We were first to detect in pathological human tear fluids the natural inhibitors of uPA, type 1 and type 2 plasminogen activator inhibitors (PAI-1 and PAI-2; Tözsér and Berta 1991). Both inhibitors belong to the serpine family, their structure and function is fairly similar, though they play different biological roles: while PAI-1 is a secreted inhibitor, PAI-2 is largely not secreted from the cells producing it (Medealf and Stasinopopulis 2005). PAI-2 expression was detected in human corneal epithelial cells (Williams et al. 1999), as well as in conjunctival cells (Massaro-Giordano et al. 2005).

Various forms of application of PAI-1 and PAI-2 are known from the art.

US Patent No. 4,923,807 and US Patent No. 5,422,090 describe PAI-2 as inhibitor of urokinase type plasminogen activator, and also disclose its potential use in malignous diseases.

US Patent No. 6,288,025 describes the possible therapeutic uses of PAI-2 (including that of recombinant PAI-2) in psoriasis.

US Patent No. 7,015,021 describes the use of a nickel-chelate chromatography in the case of PAI-1 and PAI-2 inhibitors, however it does that definitely for non-hexahistidine sequence-containing, so called natural sequences.

Zhou et al. (1997) described expression and purification of native PAI-2 from E. coli bacterial cells. In this case, however, the protein got into inclusion bodies, therefore active protein could be gained only after renaturation form 8 M urea solution.

Besides PAI-1 and its mutants, Arroyo De Prada et al. (2002) described the expression and purification of hexahistidine containing PAI-2, the one step affinity chromatography produced only fractions containing high quantities of polluting bacterial proteins.

WO/1994/005322 describes the potential use of PAI-2 for the inhibition of increased plasmin activities in cases of corneal ablations procedures. Our own studies, however, have proved the opposite role of the fibrinolytic system (Csutak et al. 2000, 2004, US Patent 7,179,461), therefore, in such cases, the application of PAI-2 would be definitely harmful and could lead to the development of corneal haze.

Petide-aldehydes were shown to be able to inhibit numerous proteases, and this inhibition depends on the peptide sequence. The tetrahedron hydrated C-terminal aldehyde group mimics the temporary state of the substrate hydrolysis (Ondetti and Cushman, 1981). Synthesis of peptide-aldehydes is known from the literature (e.g. Moulin et al. 2007), besides it is possible to have aldehydes synthesized commercially.

Various tripeptide-aldehydes have been synthesized and tested as urokinase inhibitors (Tamura et al. 2000). These inhibitors, however did not contain optimal substrate sequences. According to data in the literature (Friberger and Knös, 1979) the best specific urokinase sequence is the piro-Glu-Gly-Arg. The specificity of an inhibitor is important for the experimental applications, the more specific the substrate sequence that it is based on this, the more likely that it will block only the target enzyme and with high effectivity.

The first highly effective, synthetic, reversible thrombin inhibitor was the D-Phe-Pro-Arg-aldehyde (Ald-1), that showed significant anticoagulant activity, both in vitro and in vivo (Bajusz et al. 1975, 1978). Later various modified versions of this inhibitor were prepared (Bajusz et al. 1978). The stabilized form of this inhibitor proved to be effective in animal experiments, too (Bagdy et al. 1992).

Wang et al. (Experimental Eye Research; 2005, pages 1-8) have demonstrated that PAI-1 plays a certain role in corneal wound healing. PAI-1, however, have significant additional biological activities besides being an uPA inhibitor, which can make its applicability in human medicine questionable.

US patent No. 4.703.036 (Bajusz et al.) describes the production of peptide-aldehyde thrombin inhibitors, among others that of the D-Phe-Pro-Arg-aldehyde and also its thrombin inhibition.

US patent No. 6.121.241 (Bajusz et al.) discloses the use of D-Xaa-Pro-Arg-aldehyde as a therapeutic drug, first of all, as anticoagulant and antithrombotic agent.

The WO 00/05245 international publication pamphlet describes the synthesis of urokinase-specific aldehyde inhibitors. The biological availability of iBuOCO-D-Ser-Ala-Arg-aldehyde, described in the document, proved to be 87 % in subcutaneous application (Tamura et al. 2000).

Other potent Ald-1 derivatives were also described, e.g. the D-2-ciklohexyl-2-hydroxyacetyl-Pro-Arg-aldehyde showed very good anticoagulant and antithrombotic activity (Hungarian patent application No. 211,088; Bajusz et al. 1995).

### Summary of the invention

There is no known solution disclosed in the art, according to which any type of inhibitors of urokinase type plasminogen activator would be used for the treatment of pathological corneal disorders, such as degradative changes due to exposure to chemical agents. On the contrary, the administration of uPA into the eye proved to be effective in the treatment of certain corneal changes. The present inventors were the first to recognize that, for the treatment of certain corneal and conjunctival disorders being accompanied by pathological increase of uPA activity in the eye, the inhibition of uPA activity can be used for the prevention and treatment of the developing pathological changes.

Free urokinase that is overacting in the anterior segment of the eye is to be blocked in such a way that it should not affect the activity needed for the migration of cells. Presently there is no available solution, pharmaceutical composition or therapeutic protocol disclosed in the art that could do that.

### Detailed description of the invention

In accordance with what is described above the present invention concerns inhibitors of urokinase type plasminogen activator (uPA) for use in the prevention or treatment of corneal processes accompanied by urokinase overaction and threatening with ulceration or causing ulceration, or disorders induced by such processes.

The inhibitor of the invention is the PAI-2 protein or a fusion protein derivative thereof retaining uPA-inhibiting capacity, or the D-Phe-Pro-Arg-aldehyde (Ald-1).

The PAI-2 protein according to the invention is preferably produced by recombinant means, preferably by bacterial expression, through which expression the protein is preferably expressed together with aminoterminal sequences facilitating purification and folding.

The inhibitors of the invention are preferably used in the form of eye drops or eye ointments, preferably for at least a week, five times daily, preferably for the prevention or treatment of disorders resulting from exposure to damaging chemical agents, e.g. alkalis, particularly sodium- or calcium hydroxide.

In accordance with a preferred embodiment of the invention, the inhibitor of the invention is applied in combination with other protease inhibitors, preferably with serine-, cysteine-, or metalloprotease-inhibitors.

According to another preferred embodiment of the invention, the PAI-2 or PAI-2 fusion protein derivative inhibitor is used in the form of eye drops, in an amount of 0.1 IU/ml or higher urokinase equivalent.

The invention further concerns pharmaceutical compositions comprising the inhibitor of the urokinase type plasminogen activator according to the invention, for use in contacting them with the cornea, said pharmaceutical compositions being preferably eye drops or eye ointments. The pharmaceutical compositions of the invention comprise the PAI-2 protein or said derivative thereof retaining uPA-inhibiting capacity, or the D-Phe-Pro-Arg-aldehyde (Ald-1) as the inhibitor of the urokinase type plasminogen activator.

Pharmaceutical compositions of the invention are for use in the prevention or treatment of corneal processes accompanied by urokinase overaction and threatening with ulceration or causing ulceration, or disorders induced by such processes.

The present specification further discloses a method for producing pharmaceutical compositions comprising an inhibitor of the urokinase type of plasminogen activator, said method comprising the mixing of an active agent, having urokinase type plasminogen activator inhibitory activity, in an amount effective in the prevention or treatment of corneal disorders being accompanied by urokinase overaction and threatening with or causing corneal ulceration, with pharmaceutically acceptable carriers and/or additives. In the herein disclosed method, the PAI-2 protein or a fusion protein derivative thereof retaining uPA-inhibiting capacity, the D-Phe-Pro-Arg-aldehyde (Ald-1) is used as the active agent having inhibitory activity.

### Brief description of the figures

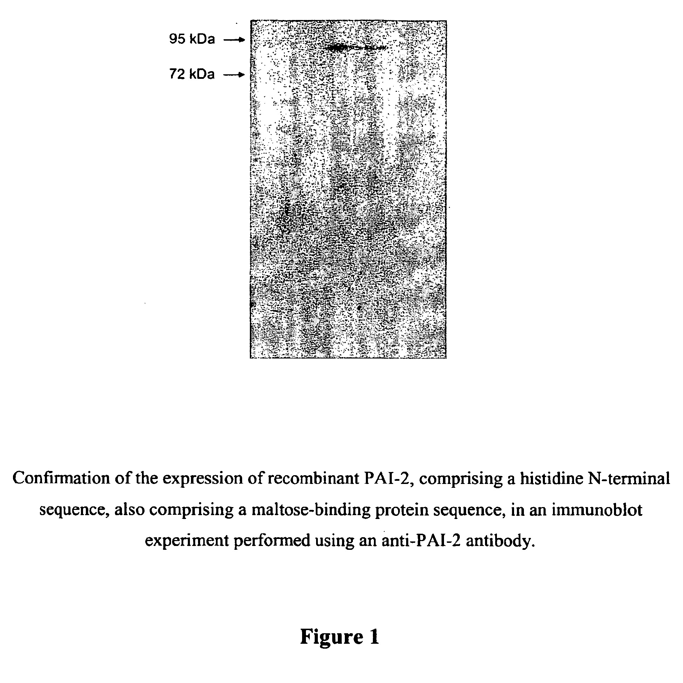

Figure 1. The confirmation of the expression of recombinant PAI-2, with histidine N-terminal sequence, that contains also maltose-binding protein sequence, using immunoblot performed with anti-PAI-2 antibody.

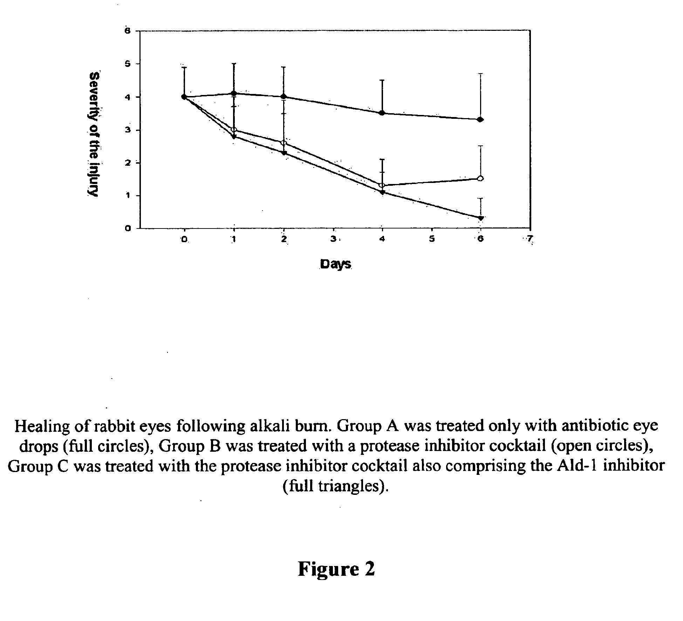

Figure 2. The healing of rabbit eyes following alkali bum. Group A was treated only with antibiotic eye drops (full circles), Group B was treated with protease inhibitor cocktail (open circles), Group C was treated with protease inhibitor cocktail also containing the Ald-1 inhibitor (full triangles).

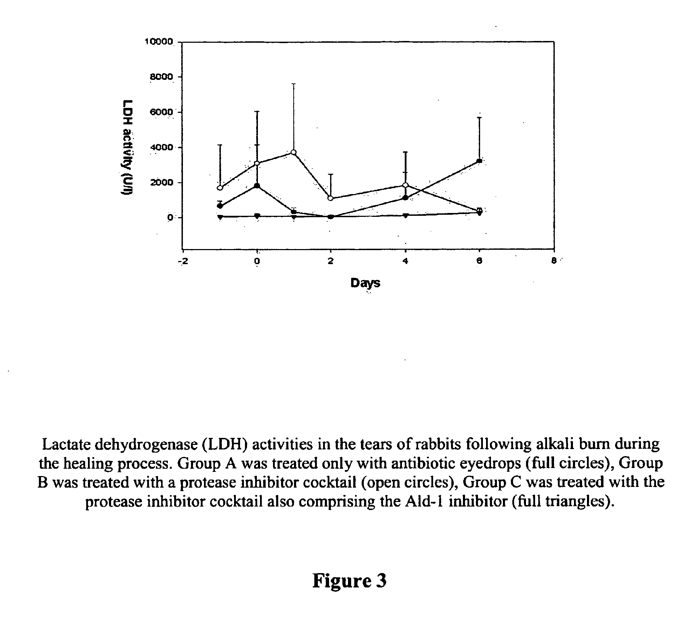

Figure 3. Lactate dehydrogenase (LDH) activities in the tears of rabbits following alkali bum during the healing process. Group A was treated only with antibiotic eye drops (full circles), Group B was treated with protease inhibitor cocktail (open circles), Group C was treated with protease inhibitor cocktail also containing the Ald-1 inhibitor (full triangles).

### Examples

The invention and its applicability will be demonstrated more closely in the following nonlimiting examples.

### Example 1.

### Production of recombinant PAI-2 fusion protein

Though PAI-1 is at least as effective as PAI-2 in blocking uPA, PAI-1 is metastable and easily takes an inactive conformation (Mottonen et al. 1992), that is unfavorable from the point of therapeutic use. The other advantage of PAI-2 is that its physiological, intracellular form does not become glycosylated, therefore the bacterially expressed protein is totally identical to the intracellular protein expressed by eukaryotic cells.

The protein producing strategy chosen by us has numerous advantages, and our disclosure is the first of producing, with such a method, recombinant protein for the purpose of use in form of eye drops. The essence of the method is hat it also comprises the use of double promoting-protein sequences. On the N-terminal of the expressed protein there is a hexahistdine sequence to help nickel-chelate chromatographic purification (Ni-NTA), which is followed by a maltose-binding sequence (MBP). Hexahistidine sequence is routinely used in protein expression to make the purification of the produced protein easier. The degree of expression is largely promoted by MBP, whereas the folding of the protein cloned behind it and its conversion to an active form is facilitated by the protein (Tropea et al. 2007). Cleavage sites of tobacco mosaic virus are cloned in between MBP and PAI-2 sequences.

For the production of recombinant protein, we created a clone that periplasmatically expresses PAI-2. Periplasm creates favorable redox surroundings for the disulphide-bridges of proteins by the presence of various disulphide isomerases. The 89 kDa fusion protein expression was performed in an Escherichia coli BL21 strain, which was grown to reach medium log phase in Luria-Bertani culture media, then the production of fusion proteins was induced by IPTG. The presence of the PAI-2 domain in the fusion protein was proved by immunoblot performed using an anti-PAI-2 antibody (Biopool). Proteins were extracted from the periplasm of the bacteria by osmotic shock procedure (Neu and Chou, 1967), then the affinity chromatography was performed on nickel-nitrilotriacetic acid (Ni-NTA) resin. Proteins were eluated by 0-150 mM linear imidasole gradient, then their purity was checked by SDS polyacrylamide gel-electrophoresis (Figure 1.). Following the removal of imidasole by dialysis, there is a possibility for the cleavage of the affinity ends by tobacco mosaic virus (TEV) protease, that also has a hexahistidine end, so with the help of a second Ni-NTA affinity chromatography the contaminations can be eliminated, and pure protein can be obtained. However, proteins were aggregated during the second step of the purification, therefore in the outflowing fluid, besides pure proteins, other components also appeared. According to our activity examinations this step was not needed, therefore in our experiments the fusion protein, concentrated by Amicon Ulta-10 kDa (millipore) concentrator, was used. The protein concentration of solutions obtained in this way was typically 0.5 mg/ml, and these solutions were directly used in the experiments.

### Example 2.

### Activity of recombinant PAI-2 fusion protein

The urokinase inhibiting activity of the protein, produced by the above described method, was tested by a microtiter plate method in the following way: 140 µl buffer (100mM Tris-HCl, 300 mM NaCl, pH=8.5), 20 µl 3 mM piro-Glu-Gly-Arg-pNA chromogenic substrate (S-2444, Chromogenix), 20 µl 700 IU/ml uPA (Choy), the activity was calibrated with S-2444 substrate in a previously described way (Tözsér and Berta, 1990), to this mixture 20 µl purified PAI-2 fusion protein (or distilled water as control) was added. The reaction was incubated for 20 minutes on 37°C, then the yellowing of the reaction mixture was checked on 405 nm with Labsystems Multiskan MS microplate reader. With this method the urokinase blocking activity of the PAI-2, prepared by us, was found to be 200 IU/ml.

The activity of the recombinant PAI-2 fusion protein was also tested on the culture of CV-40-transformed corneal epithelial cells. The cells were spread in microculturing wells (20 000 cells/well), then PBS solution containing 0.8 IU/ml or 2.0 IU/ml PAI-2 fusion protein was measured on them. After incubation for 15 minutes on 37°C the cells were repeatedly washed with PBS, then the uPA activity of the cells was measured on intact cells or following solubilization of membrane-bound uPA by adding 10 % Triton-X 100. For uPA measurements, 25 µl 2.5 mM D-Val-Leu-Lys-pNA (S-2251, Chromogenix), 25 µl plasminogen (2.5 CU/ml, Chromogenix) and 65 µl PBS was measured on the cells. The negative control contained distilled water instead of plasminogen. A dose dependent uPA activity inhibition was found, and its measure was the same with intact and solubilized cells.

### Example 3.

### The use of PAI 2 fusion protein containing eye drops in the case of experimentally induced chemical corneal injury/ulceration

Widely accepted model of corneal ulceration is the follow up of chemical burn created by Na-hydroxide in rabbit eyes (Berman et al. 1980, Wang et al., Yan et al. 2004).

To test the PAI-2 fusion protein containing eye drop, rabbit experiments were performed to model chemical burns and consecutive corneal ulceration. New Zealand white rabbits (of 3-4 kg weight) were used. Chemical bum was induced by touching the cornea for 20 sec with 6 mm diameter filter paper disc, soaked in 0.5 M NaOH. To avoid pain 1 % Tetracain eye drops were used. The rabbits did not show any sign of pain during the treatment. After the injury, the eyes were washed with physiological saline, to eliminate the remaining alkaline, which was tested by Lacmus paper, then the treatment of both eyes was performed in the following manner. The right (control) eye of the animals were treated with antibiotic (Ciloxan) eye drops 5 times daily to prevent bacterial superinfection. For the treatment of the injured left eyes 5 IU/ml uPA equivalent PAI-2 fusion protein, solved in Ciloxan eye drops, was used 5 times daily.

To determine the dose of the eye drops we determined the uPA activity, with the above described chromogenic substrate method (Csutak et al. 2003), of tear samples, collected from rabbit eyes not treated with inhibitor, that was found to be on an average 5 IU/ml, during 2-13 days following alkaline bum. Urokinase activity of normal human tears is considerably lower, than that of rabbit tears (Tözsér and Berta, 1990), and only reaches the level of 0.5-1 even in pathological cases (Tözsér et al. 1989), therefore in human use eye drops having lower PAI-2 content (0.1-1 IU/ml uPA equivalent) may also be therapeutically effective.

Table 1. shows the results of the rabbit experiments: by using PAI-2 fusion protein containing eye drops, significantly better healing results could be achieved.

### Example 4.

### In vitro urokinase inhibition experiments

As potential eye drop ingredients, two types of peptide-aldehyde molecules were tested in our examinations. The first molecule was D-Phe-Pro-Arg-aldehyde (Ald-1). D-Phe-Pro-Arg-aldehyde inhibitor was originally synthesized as a thrombin inhibitor agent (Bajusz et al. 1977), however - as both thrombin, and urokinase are arginin-specific enzymes (Friberger and Knös 1979), we supposed that it could be a good inhibitor of urokinase, too. One of the advantages of this aldehyde, is that it proved to be an effective antithrombotic drug in *in vivo* studies, and its low toxicity was also demonstrated (Bagdy et al. 1983, 1992). The other molecule tested by us was PyroGlu-Gly-Arg-aldehyde (Ald-2), that contained urokinase specific sequence.

The two aldehydes were tested in a microtiter plate method, using S-2444 Piro-Glu-Gly-Arg-pNA chromogenic substrate in the following way. The inhibitors were dissolved in distilled water in 10 mg/ml concentration, and were stored at -20 °C. Under these circumstances both inhibitors proved to be stabile. To determine the inhibitor constant 140 µl buffer (100 mM Tris-HCl, 300 mM NaCl, pH=8,5) was given to 20 µl urokinase solution (activity calibrated with substrate was 130 IU/ml, CHOAY, Paris) mixed with 20 µl inhibitors of different concentrations, then following 3 min preincubation, 20 µl S-2444 (3 mM KabiVitrum ) substrate was added to the system. The appearance of yellow color in the samples was detected fotometrically using a Labsystems Multiscan MS Microtiterplate reader. Kᵢ values derived from IC₅₀ values were found to be 5.2 nM and 4.0 nM in the case of Ald-1 and Ald-2, respectively. Thus both aldehyde compounds, studied by us, proved to be very effective inhibitors of urokinase, and, regarding their inhibitory effects, can be compared to the Kᵢ values of previously published aldehyde inhibitors (Tamura et al., 2000), moreover Ald-1 inhibited urokinase significantly more effectively than thrombin (Ki = 75 nM).

### Example 5.

### Use of aldehyde-containing eye drops in the case of experimentally induced chemical burn/ulceration

To test aldehyde-containing eye drops we performed rabbit experiments to model chemical bums and consecutive ulcerating process. New Zealand white rabbits (sizes of 2-3.5 kg) were used. The chemical bum was induced by touching the cornea with 6 mm diameter filter paper discs for 20 seconds. To avoid pain 1 % Tetracaine eye drops were used. The rabbits did not show signs of pain during the procedure. Following the chemical injury the eyes were rinsed with physiologic saline to remove the remaining alkaline, then the eyes were treated 5 times a day with the eye drops. The A eye drop (control group) contained antibiotic (Neomycin or Ciloxan, depending on the experiment) to prevent bacterial superinfection. The B group received protease-inhibitor cocktail eye drops, which inhibitors were solved in antibiotic solutions. This eye drop contained 0.2 M EDTA-t (Sigma), 0.04 M cysteine (Reanal, Budapest) and 1000 KI/ml aprotinin (Gordox, Richter Gedeon, Budapest). EDTA and cysteine were used as collagenase inhibitors (Slansky and Dohlman, 1970, Berman, et al., 1980, Salonen et al., 1987), while aprotinin as plasmin inhibitor (Salonen et al, 1987). The C group was treated with the eye drop used on the B group but the eye drops also contained 4 mM Ald-1. Taking into consideration that Ald-1 is spontaneously inactivated in aqueous solutions, this eye drop was prepared freshly daily, and stored in refrigerator between uses, knowing that under such circumstances it retains its inhibitory activity (Bajusz et al. 1990). With altered N-terminal sequence, the inactivation can be prevented (Bajusz et al. 1990).

Each group consisted of four animals. The condition of the eyes was evaluated considering the following parameters: stromal edema (0-1 points), epithelial defect, that was examined with the instillation of 1 % Na-fluorescamine eye drop (0-2 points), as well as the ulceration and the opacification of the cornea (0-2 points). The results of examinations lasting for six days are shown in Figure 2. The eyes treated in group A did not heal, ulceration and persisting opacification of the cornea developed, leading to the loss of transparency of the cornea. Group B, receiving EDTA/Aprotinin/Cysteine eye drops showed significantly faster healing, the best results, however, were seen with the use of eye drops that also contained Ald-1 inhibitor (Group C).

For biochemical characterization of the healing of the eye, we performed lactate dehydrogenase (LDH) activity measurements. LDH may get into the tears from the injured corneal epithelial cells or from inflammatory cells migrating into the wound area, and the level of its activity is proportional with the degree of healing (Kahán and Ottovay 1975). Tear samples were collected using glass capillaries, after intramuscular Pilocarpine injections (2 mg/kg), 1 day before, 1 hour after the treatment (Day 0), and on each of the following days, right before instilling the first eye drops. LDH activity was determined by UV-kinetic test (Merck, Darmstadt, Germany). The biochemical measurements confirmed that the best results were obtained using the Ald-1 containing eye drops.

**Table 1.**

| The degree of healing of the eye on the 9th day, following Na-hydroxide exposure. | | |
|---|---|---|
| Rabbit id. No | Right eye (Ciloxan) | Left eye (Ciloxan + PAI-2) |
| 73538 | +++/++++ | ++ (central part 0/+) |
| 73524 | ++++ (central part +++) | 0/+ |
| 73181 | ++ | 0/+ |
| 73591 | +++ (central part ++) | ++ (central part 0/+) |
| 73253 | +++ (central part ++) | +++ (central part +) |
| 73530 | +++ (central part ++) | ++ (central part 0/+) |

| | | |
|---|---|---|
| Corneal opacity was evaluated on a (+) - (++++) scale (subjective evaluation) | | |

### References

Arroyo De Prada, N., Schroeck, F., Sinner, E.K., Muehlenweg, B., Twellmeyer, J., Sperl, S., Wilhelm, O.G., Schmitt, M., Magdolen, V. (2002) Interaction of plasminogen activator inhibitor type-1 (PAI-1) with vitronectin. Eur. J. Biochem. 269:184-192.
Bagdy, D., Barabás, É., Diószegi, M., Bajusz, S., Fehér, A. (1983) Comparative studies on the anticoagulant effects of D-Phe-Pro-Arg-H and O-Phe-Pro-Arg-CH2Cl. Thromb. Haemostas. 50: 53.
Bagdy, D., Szabó, G., Barabás, E., Bajusz, S. (1992) Inhibition by D-MePhe-Pro-Arg-H (GYKI-14766) of thrombus growth in experimental models of thrombosis. Thromb Haemost. 68(2):125-9.
Bagdy, D., Barabás, E., Szabó, G., Bajusz, S., Széll, E. (1992) In vivo anticoagulant and antiplatelet effect of D-Phe-Pro-Arg-H and D-MePhe-Pro-Arg-H. Thromb Haemost. 67(3):357-65.
Bajusz, S., Barabás, É., Széll, E., Bagdy, D. (1975) Aldehyde inhibitors of the fibrinogen-thrombin reaction. In. Walter R, Meinhofer J, eds. Peptides: Chemistry, Structure and Biology. Ann Arbor Sci. Inc. 603-8.
Bajusz, S., Barabás, E., Tolnay, P., Széll, E., Bagdy, D. (1978) Inhibition of thrombin and trypsin by tripeptide aldehydes. Int J Pept Protein Res. 12(4):217-21.
Bajusz, S., Szell, E., Bagdy, D., Barabas, E., Horvath, G., Dioszegi, M., Fittler, Z., Szabo, G., Juhasz, A., Tomori, E., et al. (1990) Highly active and selective anticoagulants: D-Phe-Pro-Arg-H, a free tripeptide aldehyde prone to spontaneous inactivation, and its stable N-methyl derivative, D-MePhe-Pro-Arg-H. J. Med. Chem. 33(6):1729-35.
Bajusz, S., Barabás, E., Fauszt, I., Fehér, A., Horváth, G., Juhász, A., Szabó, A.G., Széll, E. (1995) Active site-directed thrombin inhibitors: alpha-hydroxyacyl-prolyl-arginals. New orally active stable analogs of D-Phe-Pro-Arg-H. Bioorg Med Chem. 3(8):1079-89.
Berman, M., Leary, R., Gage, J. (1980) Evidence for a role of the plasminogen activator--plasmin system in corneal ulceration. 1: Invest Ophthalmol Vis Sci. 19(10):1204-1221.
Barlati, S., Marchina, E., Quaranta, C.A, Vigasio, F., Semeraro, F. (1990) Analysis of fibronectin, plasminogen activators and plasminogen in tear fluid as markers of corneal damage and repair. Exp. Eye Res 51, 1-9.
Berman, M., Leary, R., Gage, J. (1980) Evidence for a role of the plasminogen activator-plasmin system in corneal ulceration. Invest. Ophthalmol. Vis. Sci. 19: 1204-21.
Crippa, M.P. (2007) Urokinase-type plasminogen activator. Int J Biochem Cell Biol. 39(4):690-694.
Croucher, D.R., Saunders, D.N., Stillfried, G.E., Ranson, M.. (2007) A structural basis for differential cell signalling by PAI-1 and PAI-2 in breast cancer cells. Biochem J. 408(2):203-210.
Csutak, A., Tözsér, J., Békési, L., Hassan, Z., Berta, A., Silver, D.M. (2000): Plasminogen activator activity in tears after Excimer Laser Photorefractive Keratectomy. Invest Ophthalmol Vis Sci. 41(12):3743-3747.
Csutak, A., Silver, D.M., Tözsér, J., Facskó, A., Berta, A. (2003): Plasminogen activator activity and inhibition in rabbit tears after photorefractive keratectomy. Exp Eye Res.77:675-680.
Csutak, A., Silver, D.M., Tözsér, J" Hassan, Z., Berta, A. (2004): Urokinase-type plasminogen activator to prevent haze after photorefractive keratectomy and pregnancy as a risk factor for haze in rabbits. Invest Ophthalmol Vis Sci. 45:1329-1333.
Friberger, P., Knös, M. (1979) Plasminogen determination in human plasma. In: Scully MF, Kakkar VV, eds. Chromogenic peptide substrates: Chemistry and clinical usage. Churchill Livingstone, 128-40.
Kahán, I.L., Ottovay, E. (1975) Lactate dehydrogenase of tears and corneal epithelium. Exp. Eye Res. 20, 129-33.
Massaro-Giordano, M., Marshall, C.M., Lavker, R.M., Jensen, P.J., Risse Marsh, B.C. (2005) Plasminogen activator inhibitor type 2 (PAI-2) is present in normal human conjunctiva. J Cell Physiol. 205(2):295-301.
Medcalf, R.L., Stasinopoulos, S.J. (2005) The undecided serpin. The ins and outs of plasminogen activator inhibitor type 2. 272(19):4858-4867.
Moulin, A., Martinez, J. and Fehrentz, J.A. (2007) Synthesis of peptide aldehydes. J. Pept. Sci. 13(1):1-15.
Ondetti, M.A., Cushman, D.W. (1981) Design of protease inhibitors. Biopolymers 20(9):2001-10.
Salonen, E.M., Tervo, T., Törma, E., Tarkkanen, A., Vaheri, A. (1987) Plasmin in tear fluid of patients with corneal ulcers: basis for new therapy. Acta Ophthalmol. 65:3-12.
Silver, D.M., Csutak, A., Berta, A. and Tözsér, J. (2007) Plasminogen activator to prevent corneal and subepithelial haze after laser vision correction surgery US Patent 7,179,461.
Slansky, H.H., Dohlman, C.H. (1970) Collagenase and the cornea. Surv. Ophthalmol. 14: 402-15.
Tamura, S.Y., Weinhouse, M.I., Roberts, C.A., Goldman, E.A., Masukawa, K., Anderson, S.M., Cohen, C.R., Bradbury, A.E., Bernardino, V.T., Dixon, S.A., Ma, M.G., Nolan, T.G. and Brunck, T.K. (2000) Synthesis and biological activity of peptidyl aldehyde urokinase inhibitors. Bioorg. Med. Chem. Lett. 10(9):983-7.
Tözsér J, Berta A, Punyiczki M. (1989) Plasminogen activator activity and plasminogen independent amidolytic activity in tear fluid from healthy persons and patients with anterior segment inflammation. Clin.Chim.Acta. 183:323-332.
Tözsér., J, Berta, A. (1990) Urokinase-type plasminogen activator in rabbit tears. Comparison with human tears. Exp. Eye Res. 51:33-7.
Tözsér, J., Berta, A., Holly, F.J. (1990): Determination of plasminogen activator activities in normal and pathological human tears. The significance of tear plasminogen activators in the inflammatory and traumatic lesions of the cornea and conjunctiva. Acta Ophthalmol. 1990 68(5):508-514.
Tözsér, J., Berta, A. (1991) Plasminogen activator inhibitors in human tears. Acta Ophthalmol. 69:426-431
Tözsér., J, Berta, A. (1990) Urokinase-type plasminogen activator in rabbit tears. Comparison with human tears. Exp. Eye Res. 51:33-37.
Tropea, J.E., Cherry, S., Nallamsetty, S., Bignon, C. and Waugh, D.S. (2007) A generic method for the production of recombinant proteins in Escherichia coli using a dual hexahistidine-maltose-binding protein affinity tag. Methods Mol. Biol. 363:1-19.
Wang, H.M., Berman, M., Law, M. (1985) Latent and active plasminogen activator in corneal ulceration. Invest Ophthalmol Vis Sci. 26(4):511-524.
Williams, D.L., Risse, B., Kim, S., Saunders, D., Orlin, S., Baker, M.S., Jensen, P.J., Lavker, R.M. (1999) Plasminogen activator inhibitor type 2 in human corneal epithelium. Invest Ophthalmol Vis Sci. 40(8):1669-1675.
Yan, J., Yang, T., Li, G., Zhang, Y., Zeng, Y., Yang, J. (2004) Changes of uPA and uPA-R expression in the cornea after alkali burn. Colloids Surf B Biointerfaces. 37(1-2):49-52.
Zhou, A., Jian, X., Dou, F., Zhu, D. and Xu, X. (1997) Renaturation, purification, and characterization of human plasminogen activator inhibitor type 2 (PAI-2) accumulated at high level in Escherichia coli. J. Biochem (Tokyo). 121 (5):930-934.

## Claims

1. An inhibitor of the urokinase type plasminogen activator (uPA) for use in the prevention or treatment of corneal processes accompanied by urokinase overaction and threatening with ulceration or causing ulceration, or disorders induced by such processes, wherein said inhibitor is the PAI-2 protein or a fusion protein derivative thereof retaining its uPA-inhibiting capacity, or the D-Phe-Pro-Arg-aldehyde (Ald-1).

2. The inhibitor for use according to claim 1, wherein said inhibitor is the PAI-2 protein or a fusion protein derivative thereof retaining its uPA-inhibiting capacity, said inhibitor protein having been produced by recombinant means, and said inhibitor protein having been expressed preferably together with aminoterminal sequences facilitating its purification and folding.

3. The inhibitor for use according to claim 1, wherein said inhibitor is the D-Phe-Pro-Arg-aldehyde (Ald-1).

4. The inhibitor for use according to any preceding claim, wherein said inhibitor is in the form of an eye drop or eye ointment.

5. The inhibitor for use according to any preceding claim, wherein said disorders have developed as a consequence of an exposure of the cornea to a damaging chemical agent.

6. The inhibitor for use according to claim 5, wherein said disorders have developed as a consequence of the exposure of the cornea to an alkali.

7. The inhibitor for use according to claim 6, wherein said disorders have developed as a consequence of the exposure of the cornea to sodium or calcium hydroxide.

8. The inhibitor for use according to any preceding claim, wherein said inhibitor is used in combination with other protease inhibitors.

9. The inhibitor for use according to claim 8, wherein said other inhibitors are serine-, cysteine- or metalloprotease inhibitors.

10. The inhibitor for use according to claim 2, wherein said inhibitor is in the form of an eye drop, in an amount of 0.1 IU/ml or higher urokinase equivalent.

11. A pharmaceutical composition comprising a specific inhibitor of the urokinase type plasminogen activator, wherein said plasminogen activator inhibitor is the PAI-2 protein or a fusion protein derivative thereof retaining uPA-inhibiting capacity, or the D-Phe-Pro-Arg-aldehyde (Ald-1), for use in contacting it with the cornea.

12. A pharmaceutical composition for use according to claim 11, wherein said composition is in the form of an eye drop or an eye ointment.

13. The pharmaceutical composition of claim 11, for use in the prevention or treatment of corneal processes accompanied by urokinase overaction and threatening with ulceration or causing ulceration, or disorders induced by such processes.

## Patentansprüche

1. Ein Inhibitor des Plasminogen Aktivators vom Urokinasetyp (uPA) zur Verwendung beim Verhindern oder Behandeln von die Hornhaut betreffenden Prozessen, die von einer Urokinase-Überaktion begleitet werden und bei denen die Gefahr einer Bildung eins Geschwürs besteht oder die eine Bildung eines Geschwürs bewirken, oder von Funktionsstörungen, die durch derartige Prozesse induziert werden, wobei der Inhibitor das PAI-2-Protein oder ein Fusionsproteinderivat davon, das seine uPA-Inhibierungsfähigkeit behält, oder das D-Phe-Pro-Arg-Aldehyd (Ald-1) ist.

2. Der Inhibitor zur Verwendung gemäß Anspruch 1, wobei der Inhibitor das PAI-2-Protein oder ein Fusionsproteinderivat davon ist, das seine uPA-Inhibierungsfähigkeit behält, wobei das Inhibitorprotein durch rekombinante Mittel erzeugt wurde und das Inhibitorprotein vorzugsweise zusammen mit aminoterminalen Sequenzen exprimiert wurde, die seine Reinigung und Faltung erleichtern.

3. Der Inhibitor zur Verwendung gemäß Anspruch 1, wobei der Inhibitor das D-Phe-Pro-Arg-Aldehyd (Ald-1) ist.

4. Der Inhibitor zur Verwendung gemäß irgendeinem der vorhergehenden Ansprüche, wobei der Inhibitor in Form von Augentropfen oder einer Augensalbe vorliegt.

5. Der Inhibitor zur Verwendung gemäß irgendeinem der vorhergehenden Ansprüche, wobei sich die Funktionsstörungen als eine Folge dessen entwickelt haben, dass die Hornhaut einem schädlichen chemischen Stoff ausgesetzt wurde.

6. Der Inhibitor zur Verwendung gemäß Anspruch 5, wobei sich die Funktionsstörungen als eine Folge dessen entwickelt haben, dass die Hornhaut einer Lauge ausgesetzt wurde.

7. Der Inhibitor zur Verwendung gemäß Anspruch 6, wobei sich die Funktionsstörungen als eine Folge dessen entwickelt haben, dass die Hornhaut Natrium- oder Calciumhydroxid ausgesetzt wurde.

8. Der Inhibitor zur Verwendung gemäß irgendeinem der vorhergehenden Ansprüche, wobei der Inhibitor in Kombination mit anderen Proteaseinhibitoren verwendet wird.

9. Der Inhibitor zur Verwendung gemäß Anspruch 8, wobei die anderen Inhibitoren Serin-, Cystein- oder Metalloprotease-Inhibitoren sind.

10. Der Inhibitor zur Verwendung gemäß Anspruch 2, wobei der Inhibitor in Form von Augentropfen in einer Menge von 0,1 IU / ml oder einem höheren Urokinaseäquivalent ist.

11. Eine pharmazeutische Zusammensetzung, umfassend einen spezifischen Inhibitor des Plasminogen Aktivators vom Urokinasetyp, wobei der Inhibitor des Plasminogen Aktivators das PAI-2-Protein oder ein Fusionsproteinderivat davon, das eine uPA-Inhibierungsfähigkeit beibehält, oder das D-Phe-Pro-Arg-Aldehyd (Ald-1) ist, zur Verwendung beim Kontaktieren derselben mit der Hornhaut.

12. Eine pharmazeutische Zusammensetzung zur Verwendung gemäß Anspruch 11, wobei die Zusammensetzung in Form von Augentropfen oder einer Augensalbe vorliegt.

13. Die pharmazeutische Zusammensetzung gemäß Anspruch 11 zur Verwendung beim Verhindern oder Behandeln von die Hornhaut betreffenden Prozessen, die von einer Urokinase-Überaktion begleitet werden und bei denen die Gefahr einer Bildung eines Geschwürs besteht oder die eine Bildung eines Geschwürs verursachen, oder von Funktionsstörungen, die durch derartige Prozesse induziert werden.

## Revendications

1. Inhibiteur de l'activateur du plasminogène de type urokinase (uPA) pour une utilisation dans la prévention ou le traitement de processus cornéens accompagnés d'une action excessive de l'urokinase et d'une menace d'ulcération ou de la provocation d'une ulcération, ou de troubles induits par ces processus, où ledit inhibiteur est la protéine PAI-2 ou un dérivé de protéine de fusion de celle-ci conservant sa capacité d'inhibition de l'uPA, ou le D-Phe-Pro-Arg-aldéhyde (Ald-1).

2. Inhibiteur pour une utilisation selon la revendication 1, où ledit inhibiteur est la protéine PAI-2 ou un dérivé de protéine de fusion de celle-ci conservant sa capacité d'inhibition de l'uPA, ladite protéine inhibitrice ayant été produite par des techniques de recombinaison, et ladite protéine inhibitrice ayant été exprimée de préférence conjointement avec des séquences amino-terminales facilitant sa purification et son repliement.

3. Inhibiteur pour une utilisation selon la revendication 1, où ledit inhibiteur est le D-Phe-Pro-Arg-aldéhyde (Ald-1).

4. Inhibiteur pour une utilisation selon l'une quelconque des revendications précédentes, où ledit inhibiteur est sous la forme de gouttes pour les yeux ou d'une pommade pour les yeux.

5. Inhibiteur pour une utilisation selon l'une quelconque des revendications précédentes, où lesdits troubles se sont développés comme conséquence d'une exposition de la cornée à un agent chimique dommageable.

6. Inhibiteur pour une utilisation selon la revendication 5, où lesdits troubles se sont développés comme conséquence de l'exposition de la cornée à un alcali.

7. Inhibiteur pour une utilisation selon la revendication 6, où lesdits troubles se sont développés comme conséquence de l'exposition de la cornée à de l'hydroxyde de sodium ou de calcium.

8. Inhibiteur pour une utilisation selon l'une quelconque des revendications précédentes, où ledit inhibiteur est utilisé en combinaison avec d'autres inhibiteurs des protéases.

9. Inhibiteur pour une utilisation selon la revendication 8, où lesdits autres inhibiteurs sont des inhibiteurs de sérine-, cystéine- ou métallo-protéases.

10. Inhibiteur pour une utilisation selon la revendication 2, où ledit inhibiteur est sous la forme de gouttes pour les yeux, dans une quantité de 0,1 UI/ml ou supérieure d'équivalent d'urokinase.

11. Composition pharmaceutique comprenant un inhibiteur spécifique de l'activateur du plasminogène de type urokinase, où ledit inhibiteur de l'activateur du plasminogène est la protéine PAI-2 ou un dérivé de protéine de fusion de celle-ci conservant sa capacité d'inhibition de l'uPA, ou le D-Phe-Pro-Arg-aldéhyde (Ald-1), pour une utilisation le mettant en contact avec la cornée.

12. Composition pharmaceutique pour une utilisation selon la revendication 11, où ladite composition est sous la forme de gouttes pour les yeux ou d'une pommade pour les yeux.

13. Composition pharmaceutique selon la revendication 11, pour une utilisation dans la prévention ou le traitement de processus cornéens accompagnés d'une action excessive de l'urokinase et d'une menace d'ulcération ou de la provocation d'une ulcération, ou de troubles induits par ces processus.
